Europäisches Patentamt
European Patent Office
Office européen des brevets

(19) (11) Veröffentlichungsnummer: 0 245 690
A1

(12) EUROPÄISCHE PATENTANMELDUNG

(21) Anmeldenummer: 87106123.0

(22) Anmeldetag: 28.04.87

(51) Int. Cl.³: C 07 D 215/56
C 07 C 101/34, C 07 C 69/73-8

(30) Priorität: 10.05.86 DE 3615767

(43) Veröffentlichungstag der Anmeldung: 19.11.87 Patentblatt 87/47

(84) Benannte Vertragsstaaten: AT BE CH DE ES FR GB IT LI NL SE

(71) Anmelder: BAYER AG
Konzernverwaltung RP Patentabteilung
D-5090 Leverkusen 1 Bayerwerk(DE)

(72) Erfinder: Schriewer, Michael, Dr.
Am Thelen Siefen 1a
D-5068 Odenthal(DE)

(72) Erfinder: Grohe, Klaus, Dr.
Am Wasserturm 10
D-5068 Odenthal(DE)

(54) **Verfahren zur Herstellung von 4-Hydroxy-chinolin-3-carbon-säuren.**

(57) Die Erfindung betrifft ein Verfahren zur Herstellung von 4-Hydroxy-chinolin-3-carbonsäuren der allgemeinen Formel (I)

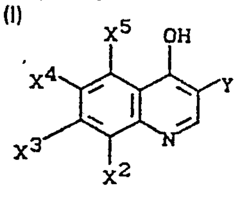

I

in welcher Y, $X^2$, $X^3$, $X^4$ und $X^5$ die in der Beschreibung angegebenen Bedeutungen besitzen. Diese Verbindungen sind wichtige Zwischenprodukte, insbesondere zur Darstellung antibakteriell wirksamer 1-Alkyl-4-chinolon-3-carbonsäuren.

EP 0 245 690 A1

0245690

BAYER AKTIENGESELLSCHAFT
Konzernverwaltung RP
Patentabteilung
5090 Leverkusen, Bayerwerk
Ad/ABc

# Verfahren zur Herstellung von 4-Hydroxy-chinolin-3-carbonsäuren

Die Erfindung betrifft ein neues Verfahren zur Herstellung von 4-Hydroxy-chinolin-3-carbonsäuren. Diese Verbindungen sind wichtige Zwischenprodukte, insbesondere zur Darstellung antibakteriell wirksamer 1-Alkyl-4-chinolon-3-carbonsäuren.

Die Darstellung von 4-Hydroxy-chinolin-3-carbonsäuren ist bekannt (Prog. Drug Research 21 (1977), 9).

Sie werden erhalten, wenn man entsprechend substituierte Aniline mit Alkoxymethylenmalonestern umsetzt und das Primäraddukt entweder durch Erhitzen in Polyphosphorsäure oder in Diphenylether cyclisiert.

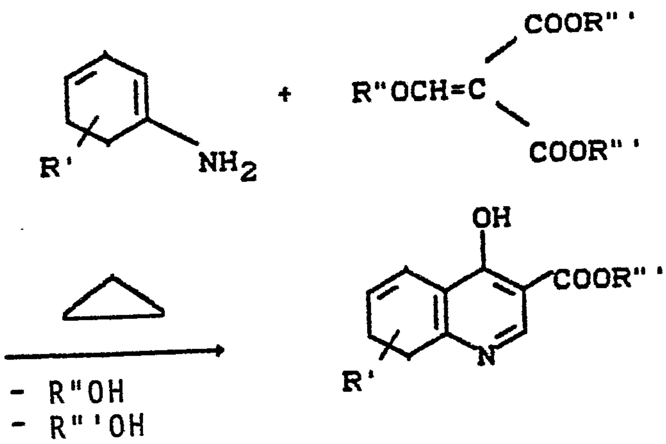


Dieses Verfahren hat jedoch einige wesentliche Nachteile:

1) Es sind hohe Reaktionstemperaturen erforderlich. Dies ist besonders nachteilig bei thermolabilen Substituenten.

2) Je nach Substitution am Anilin ist mit einem Gemisch isomerer Chinoline zu rechnen.

3) Mit elektronenziehenden Substituenten bestückte Aniline reagieren schlecht.

Im Gegensatz dazu führt das erfindungsgemäße Verfahren unter milden Bedingungen und in guten Ausbeuten zu isomerenreinen 4-Hydroxy-chinolin-3-carbonsäuren. Demnach können Verbindungen der Formel I

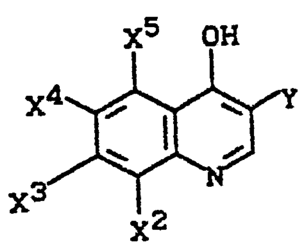
I

in welcher

Y eine Nitrilgruppe, eine Estergruppe $COOR^1$ oder ein Säureamid $CONR^2R^3$ darstellt, wobei $R^1$, $R^2$ und $R^3$ für Wasserstoff oder $C_1$-$C_4$-Alkyl stehen und $R^3$ gegebenenfalls Phenyl sein kann und

$X^2$, $X^3$, $X^4$ und $X^5$ für Wasserstoff, Halogen, Nitro, Cyano, Alkyl mit 1-3 Kohlenstoffatomen, Alkoxy mit 1-3 Kohlenstoffatomen im Alkylrest, Alkylmercapto mit 1-3 Kohlenstoffatomen im Alkylrest, Alkylsulfonyl mit 1-3 Kohlenstoffatomen im Alkylrest, sowie eine gegebenenfalls im Arylrest substituierte Phenylsulfonylgruppe stehen, erhalten werden, wenn man Aminoacrylester der Formel II

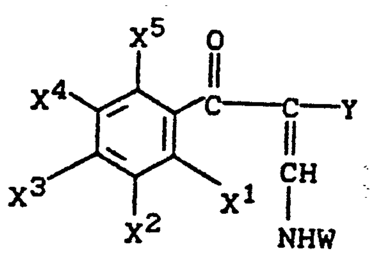

II

in der Y, $X^2$, $X^3$, $X^4$ und $X^5$ die oben angegebene Bedeutung haben, und

$X^1$ Halogen, bevorzugt Fluor und Chlor, eine Nitrogruppe, eine Alkoxy-, Alkylmercapto- oder Alkylsulfonylgruppe mit jeweils 1-3 Kohlenstoffatomen sowie eine Arylsulfonylgruppe bedeutet und

W für Wasserstoff und einen Rest $-CH_2CH_2Z$ steht, wobei

Z für eine Nitrilgruppe, eine Estergruppe $COOR^4$ oder eine Säureamidgruppe $CONR^5R^6$ steht, wobei die Reste $R^4$, $R^5$, $R^6$ Wasserstoff oder $C_1$-$C_4$-Alkyl darstellen und $R^6$ gegebenenfalls Phenyl sein kann,

in einem aprotischen Lösungsmittel in Gegenwart einer Base umsetzt.

Dabei ist es als überraschend zu bezeichnen, daß im Falle $W = -CH_2CH_2Z$ die Cyclisierung von II und die Abspaltung der Hilfsgruppe $CH_2=CHZ$ unter Bildung von I in einer sogenannten Eintopfreaktion ablaufen.

Die als Ausgangsstoffe für das erfindungsgemäße Verfahren benötigten Enamine II können durch Umsetzung der Enolether der Formel III

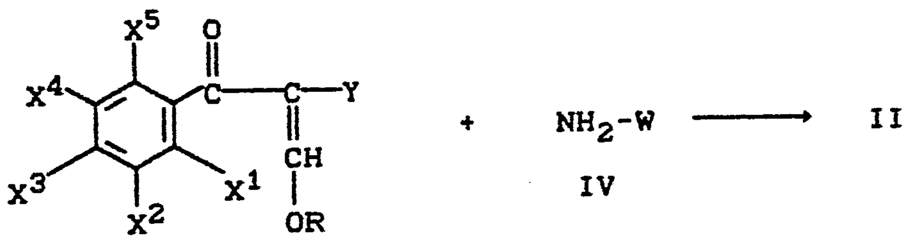

III

in der $X^1$-$X^5$ und Y die angegebene Bedeutung haben, mit Aminen der Formel IV, in der W die oben angegebene Bedeutung hat, erhalten werden.

Die Enolether III sind bekannt oder können gemäß folgendem allgemeinen Reaktionsschema hergestellt werden:

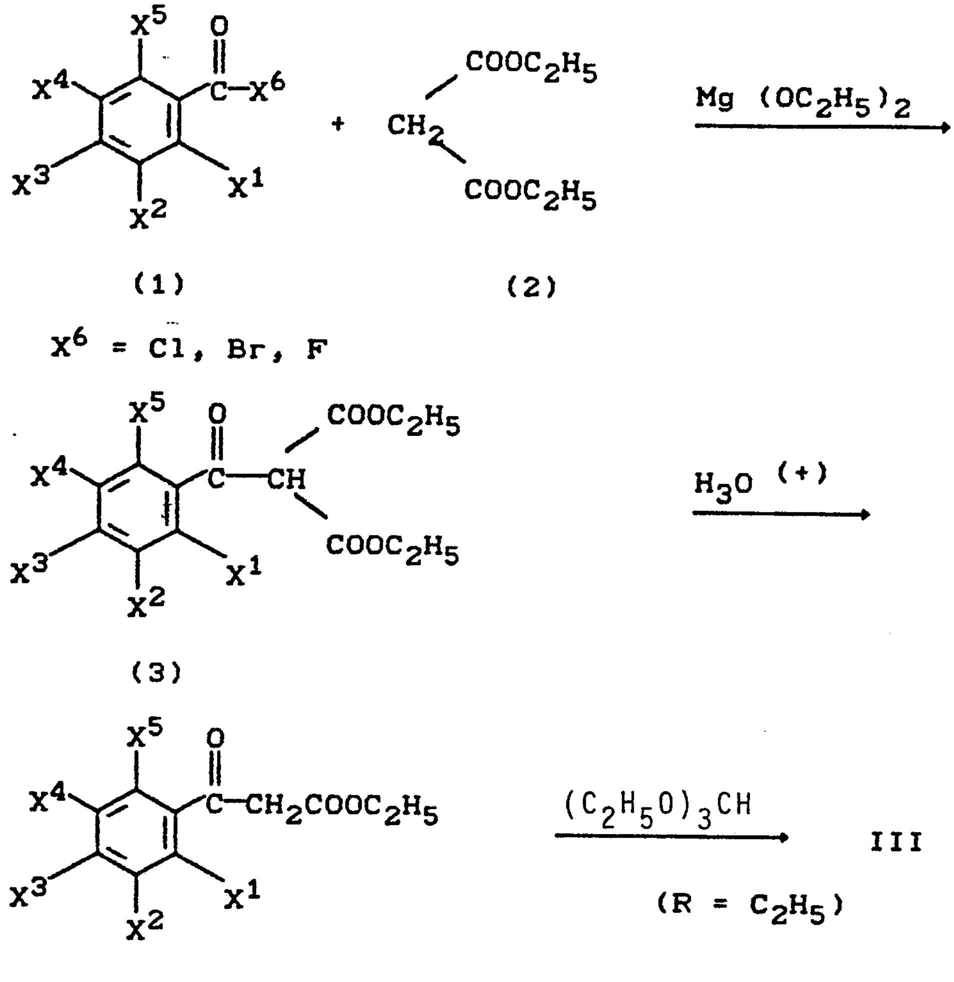


(1) (2)

$X^6$ = Cl, Br, F

(3)

(4)

Danach wird Malonsäurediethylester (2) in Gegenwart von Magnesiumethylat mit dem entsprechenden Benzoylhalogenid (1) zum Acylmalonester (3) acyliert (Organicum, 3. Aufl. 1964, S. 438).

Durch partielle Verseifung und Decarboxylierung von (3) in wäßrigem Medium mit katalytischen Mengen Schwefelsäure oder 4-Toluolsulfonsäure erhält man in guter Ausbeute den Benzoylessigsäureethylester (4), der mit Orthoameisensäure-triethylester/Acetanhydrid in den 2-Benzoyl-3-ethoxy-acrylsäureethylester (III R = $C_2H_5$) übergeht. Die Umsetzung von (III) mit den Aminen (IV) in einem Lösungsmittel wie z.B. Methylenchlorid, einem Alkohol, Chloroform, Cyclohexan oder Toluol führt in leicht exothermer Reaktion zu den gewünschten Zwischenprodukten (II).

Die Cyclisierungsreaktionen (II) ⟶ (I) werden in einem Temperaturbereich von etwa 60 - 300°C, bevorzugt 80 - 180°C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Die Cyclokondensationen können bei Normaldruck, aber auch bei erhöhtem Druck durchgeführt werden. Im allgemeinen arbeitet man bei Drucken zwischen etwa 1 und etwa 100 bar, vorzugsweise 1 und 10 bar.

Als Säurebinder kommen für die Cyclisierungsreaktionen (II) ⟶ (I) Kalium-tert.-butanolat, 1,4-Diaza-bicyclo[2,2,2]-octan (DABCO), 1,8-Diaza-bicyclo[5,4,0]undec-7-en (DBU), Butyl-lithium, Lithium-phenyl, Phenyl-magnesiumbromid, Natriummethylat, Natriumethylat, Natriumhydrid, Natrium- oder Kaliumcarbonat in Betracht. Besonders bevorzugt werden Kalium-oder Natriumfluorid, wenn Fluorwasserstoff abgespalten werden muß.

Für die Cyclisierung (II) ⟶ (I) werden im Fall W = Wasserstoff zwei Äquivalente Base, im Fall W = $CH_2CHZ$ ein Äquivalent einer Base benötigt. Es kann vorteilhaft sein, jeweils einen Überschuß von 10 Mol-% Base einzusetzen.

Die als Ausgangsstoffe für diesen Syntheseweg verwendeten Benzoylhalogenide (1) sind bekannt. Als Beispiele seien genannt: 2,3,4,5-Tetrafluorbenzoylchlorid, Pentafluorbenzoylchlorid, 2,4-Dichlor-5-fluorbenzoylchlorid, 2,4-Dichlorbenzoylchlorid, 2-Chlor-5-fluorbenzoylchlorid, 2-Chlor-5-fluorbenzoylchlorid, 2-Chlor-4-methyl-benzoylchlorid, 2,4,5-Trifluorbenzoylfluorid, 2,5-Dichlorbenzoylfluorid, 2,3,4,5-Tetrachlorbenzoylchlorid, 4-Nitro-2-Chlor-benzoylchlorid.

Die als Ausgangsstoffe für diesen Syntheseweg verwendeten Amine der Formel IV sind bekannt. Als Beispiele seien genannt:

Ammoniak, 3-Aminopropionsäurenitril, 3-Aminopropionsäureethylester, 3-Aminopropionsäuredimethylamid, 3-Aminpropionsäureanilid.

Weiterer Gegenstand der Erfindung sind 4-Hydroxy-3-chinolincarbonsäurederivate der Formel I

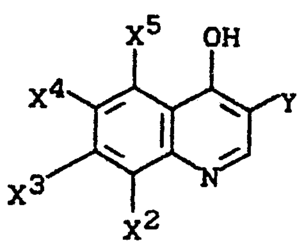

I

in welcher

Y und $X^5$ die oben angegebene Bedeutung haben und

$X^2$ für Nitro, Cyano, eine Alkyl-, Alkoxy-, Alkylmercapto- oder Alkylsulfonylgruppe mit jeweils 1-3 Kohlenstoffatomen im Alkylteil sowie eine gegebenenfalls im Arylrest substituierte Phenyl sulfonylgruppe steht und

$X^3$ und $X^4$ für Nitro und Halogen, insbesondere Chlor und Fluor stehen.

Ebenfalls zum Gegenstand der Erfindung gehören Verbindungen der Formel I, in denen

Y, $X^2$, $X^3$, $X^4$ und $X^5$ die oben angegebene Bedeutung haben und
$X^2$, $X^3$, $X^4$ und $X^5$ zusätzlich für Amino stehen können.

Diese Verbindungen können durch literaturbekannte Reduktionsmethoden aus den entsprechenden Nitro-Derivaten erhalten werden.

Die folgenden Beispiele erläutern die Erfindung:

Beispiel 1

2-(2,4-Dichlor-5-fluor-3-nitro-benzoyl)-3-ethoxy-acrylsäure-ethylester

a) 2,4-Dichlor-5-fluor-3-nitro-benzoesäure

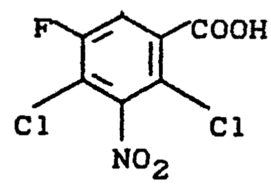

Unter Eiskühlung und Rühren werden 34 ml konz. Schwefelsäure tropfenweise mit 40 ml konz. Salpetersäure versetzt. In dieses Nitriergemisch trägt man portionsweise 20,9 g 2,4-Dichlor-5-fluorbenzoesäure ein, wobei die Temperatur auf 45 - 50°C steigt. Dann wird noch 3 Stunden auf 90 - 100°C erhitzt, das auf Raumtemperatur abgekühlte Gemisch auf 350 ml Eiswasser gegossen, der Niederschlag abgesaugt und mit Wasser gewaschen. Das feuchte Rohprodukt wurde in 30 ml Methanol heiß gelöst und die Lösung mit 150 ml $H_2O$ versetzt. Der Niederschlag wird kalt abgesaugt, mit $CH_3OH/H_2O$ gewaschen und im Vakuum bei 80°C getrocknet. Es werden 21,2 g rohe 2,4-Dichlor-5-fluor-3-nitro-benzoesäure erhalten. Sie ist genügend rein für die weiteren Umsetzungen. Eine Probe aus Toluol/Petrolether umkristallisiert liefert Kristalle vom Schmelzpunkt 192°C.

b) 2,4-Dichlor-5-fluor-3-nitro-benzoylchlorid

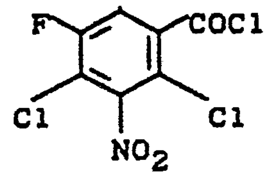

106,6 g 2,4-Dichlor-5-fluor-3-nitro-benzoesäure werden mit 250 ml Thionylchlorid 2 Stunden unter Rückfluß zum Sieden erhitzt. Das überschüssige Thionylchlorid wird dann bei Normaldruck abdestilliert und der Rückstand im Feinvakuum fraktioniert. Bei 110 -115° C/0,08 -0,09 mbar gehen 104,7 g 2,4-Dichlor-5-fluor-3-nitro-benzoylchlorid über. Beim Stehen bilden sich Kristalle vom Schmelzpunkt 35-37° C.

c) (2,4-Dichlor-5-fluor-3-nitro-benzoyl)-essigsäure-ethylester

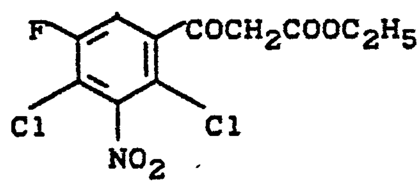

10,1 g Magnesiumspäne werden in 21 ml Ethanol mit 2,1 g Tetrachlormethan versetzt und nach Beginn der Wasserstoff-Entwicklung ein Gemisch aus 66,6 g Malonsäurediethylester, 40 ml Ethanol und 150 ml Toluol bei 50 - 60° C tropfenweise zugefügt. Man rührt 1 Stunde bei dieser Temperatur nach, kühlt auf -5 - -10° C und tropft langsam eine Lösung von 109,2 g 2,4-Dichlor-5-fluor-3-nitro-benzoylchlorid in 50 ml Toluol zu. Danach wird 1 Stunde bei 0° C gerührt, über

Nacht auf Raumtemperatur gebracht und noch 2 Stunden auf 40-50° C erwärmt. Das Reaktionsgemisch wird unter Eiskühlung mit einem Gemisch aus 160 ml Wasser und 10,4 ml konz. Schwefelsäure versetzt und die organische Phase abgetrennt. Die wäßrige Phase wird mit Toluol extrahiert und der vereinigte organische Extrakt mit gesättigter Natriumchlorid-Lösung gewaschen, mit Natriumsulfat getrocknet und das Lösungsmittel abgezogen. Man erhält 144,5 g (2,4-Dichlor-5-fluor-3-nitro-benzoyl)malonsäurediethylester als Rohprodukt. Dieses wird nach Zugabe von 200 ml Wasser und 0,6 g 4-Toluolsulfonsäure 3 Stunden unter Rückfluß erhitzt, die Mischung mit Methylenchlorid extrahiert, der Extrakt mit Natriumsulfat getrocknet und das Lösungsmittel im Vakuum abdestilliert. Es werden 118 g substituierter Benzoylessigester als Rohprodukt erhalten. Er besitzt eine für die weiteren Umsetzungen genügende Reinheit.

d) 2-(2,4-Dichlor-5-fluor-3-nitro-benzoyl)-3-ethoxy-acrylsäureethylester

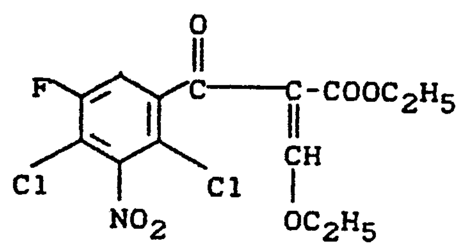

244,8 g (2,4-Dichlor-5-fluor-3-nitro-benzoyl)-essigsäureethylester werden mit 166 g Orthoameisensäuretriethylester und 185 g Essigsäureanhydrid 3 Stunden

auf 150 -160° C erhitzt. Man engt dann im Vakuum ein und erhält 270 g Benzoyl-ethoxy-acrylsäure-ethylester als öligen Rückstand.

Beispiel 2

2-(2,4-Dichlor-5-fluor-3-methylbenzoyl)-3-ethoxy-acrylsäureethylester

a) 2,4-Dichlor-3-methyl-5-nitrobenzoesäure

30 g 2,4-Dichlor-3-methylbenzoesäure werden in 83 ml konz. $H_2SO_4$ vorgelegt. Unter Eiskühlung werden 16,7 g $KNO_3$ portionsweise zugegeben. Man erwärmt anschließend noch 2 Stunden auf 50° C und gießt dann auf Eis. Die Nitroverbindung wird isoliert und aus Toluol umkristallisiert. Fp. 152-4° C, Ausbeute: 24 g.

b) 5-Amino-2,4-dichlor-3-methylbenzoesäure

55 g 2,4-Dichlor-3-methyl-5-nitrobenzoesäure und 141,6 g $Na_2S_2O_4$ werden in einem Gemisch aus 440 ml Glykolmonomethylether und 440 ml Wasser 3 Stunden gekocht. Zu der noch warmen Lösung gibt man 620 ml 1/2 konz. HCl und kocht dann noch einmal auf. Nach Abkühlen auf Raumtemperatur gießt man in 1,5 l Wasser und stellt mit Soda auf pH 5 ein. Es werden 24,6 g Aminobenzoesäure erhalten. Fp. 202-3° C.

c) 5-Amino-2,4-dichlor-3-methylbenzoesäure-methylester

24 g 5-Amino-2,4-dichlor-3-methylbenzoesäure werden in 100 ml Methanol vorgelegt. Man leitet 20 Minuten HCl-Gas ein und kocht anschließend 5 Stunden am Rückfluß. Danach gießt man in Wasser und stellt mit Soda alkalisch. Es werden 24 g Ester vom Fp. 86-88°C erhalten.

d) 2,4-Dichlor-5-fluor-3-methylbenzoesäure

24 g 5-Amino-2,4-dichlor-3-methyl-benzoesäuremethylester werden in wäßriger Lösung mit $NaNO_2$/HCl diazotiert. Zu der Diazoniumsalzlösung gibt man bei 0° C 26 ml 30 %ige wäßrige $HBF_4$-Lösung. Das Reaktionsgemisch wird 30 Minuten bei 0° C gehalten. Danach isoliert man das Tetrafluoroborat und trocknet es über $P_2O_5$. Ausbeute 23,5 g.

68 g dieses Tetrafluoroborates werden in o-Dichlorbenzol thermisch zersetzt. Nach Aufarbeitung werden 15 g 2,4-Dichlor-5-fluor-3-methylbenzoesäuremethylester vom Siedepunkt 139-40° C (16 mbar) erhalten.

12,8 g dieses Esters werden mit 4 g NaOH in 100 ml Ethanol/Wasser 1 1/2 Stunden gekocht. Nach Abkühlen wird mit HCl angesäuert und abgenutscht. Es werden 12 g der Titelverbindung erhalten.
Schmelzpunkt 167-69° C.

e) (2,4-Dichlor-5-fluor-3-methyl-benzoyl)-essigsäureethylester

Zu 1,4 g Magnesiumspänen werden 3 ml Ethanol und 0,5 ml Tetrachlorkohlenstoff gegeben. Nach Anspringen der Reaktion gibt man bei 50° C eine Mischung von 8,7 g Malonsäurediethylester, 6 ml Ethanol und 23 ml Toluol zu. Man erwärmt 1 Stunde auf 50 - 60° C und kühlt dann auf -5 - -10° C ab. Bei dieser Temperatur wird eine Lösung von 13,3 g 2,4-Dichlor-5-fluor-3-methylbenzoesäurechlorid (aus 2,4-Dichlor-5-fluor-3-methylbenzoesäure und Thionylchlorid) in 6 ml Toluol zugetropft. Danach läßt man auf Raumtemperatur kommen. Nach Stehen über Nacht wird ein Gemisch aus 24 ml Wasser und 4 ml $H_2SO_4$ zugegeben. Man trennt die Phasen, wäscht die org. Phase mit Wasser, trocknet und engt ein. Zum öligen Rückstand fügt man 20 ml Wasser und 0,1 g p-Toluolsulfonsäure. Man kocht 4,5 Stunden am Rückfluß, trennt anschließend die Phasen, trocknet die org. Phase und engt sie im Vakuum ein. Es bleiben 14,1 g der Titelverbindung als rohes Öl zurück.

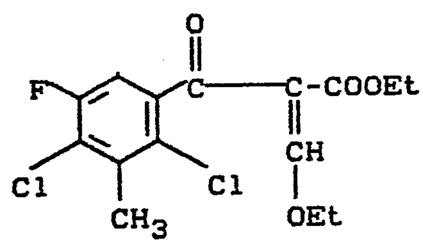

f) 2-(2,4-Dichlor-5-fluor-3-methyl-benzoyl)-3-ethoxy-acrylsäureethylester

14,1 g Verbindung aus e), 10,7 g Orthoameisensäureethylester und 12,3 g Essigsäureanhydrid werden 2 Stunden gekocht. Danach wird zunächst im Wasserstrahlvakuum und dann im Hochvakuum bis 130° C Badtemperatur abdestilliert.
Es bleiben 14 g der Titelverbindung zurück (Öl).

Beispiel 3

2-(2,4-Difluor-3-methyl-benzoyl)-3-ethoxyacrylsäure-ethylester

a) 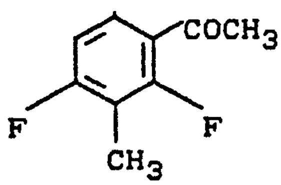 2,4-Difluor-3-methyl-acetophenon

In eine Suspension von 48 g $AlCl_3$ in 120 ml 1,2-Dichlorethan werden unter Kühlung zunächst 24,6 g Acetylchlorid und anschließend 38,5 g 2,6-Difluortoluol getropft. Danach wird 2,5 Stunden bei 50° C gerührt. Anschließend gibt man das Reaktionsgemisch auf Eis, trennt die Phasen und extrahiert die wäßrige Phase mit 1,2-Dichlorethan. Die vereinigten org. Phasen werden mit NaOH und Wasser gewaschen, über $K_2CO_3$ getrocknet und eingeengt. Der Rückstand wird unter vermindertem Druck destilliert.
Sdp.: 90 - 93° C (10 Torr)
Ausbeute: 42,9 g

b) 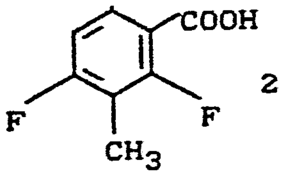 2,4-Difluor-3-methyl-benzoesäure

In eine unter Eiskühlung aus 72 g NaOH, 86,4 g Brom und 360 ml Wasser bereitete Bromlauge wird bei 10°C eine Lösung aus 30 g 2,4-Difluor-3-methylacetophenon in 270 ml Dioxan getropft. Man läßt auf Raumtemperatur kommen, fügt noch 80 ml Wasser zu und trennt die Phasen. Die wäßrige Phase wird mit einer Lösung von 18 g $Na_2S_2O_5$ in 270 ml Wasser versetzt. Danach wird mit HCl sauer gestellt, wobei die Säure ausfällt. Ausbeute 24,9 g 2,4-Difluor-3-methylbenzoesäure Schmelzpunkt 180-81°C.

c) 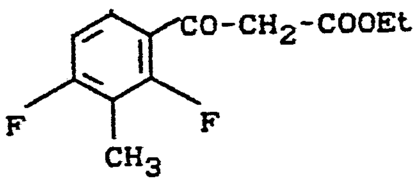

(2,4-Difluor-3-methyl-benzoyl)-essigsäureethylester

Zu 3,6 g Magnesiumspänen werden 8 ml Ethanol und 1 ml Tetrachlorkohlenstoff gegeben. Nach Anspringen der Reaktion gibt man bei 50°C eine Mischung von 22,2 g Malonsäureethylester, 15 ml Ethanol und 60 ml Toluol zu. Man erwärmt 1 Stunde auf 50-60°C und kühlt dann auf -5 - -10°C ab. Bei dieser Temperatur wird eine Lösung von 25,7 g 2,4-Difluor-3-methyl-benzoesäurechlorid (aus 2,4-Difluor-3-methylbenzoesäure und Thionylchlorid) in 15 ml Toluol zugetropft. Danach

läßt man auf Raumtemperatur kommen. Nach Stehen über Nacht wird ein Gemisch von 60 ml Wasser und 10 ml $H_2SO_4$ zugetropft. Man trennt die Phasen, wäscht die org. Phase mit Wasser, trocknet und engt ein. Zum öligen Rückstand fügt man 50 ml Wasser und 0,2 g p-Toluolsulfonsäure. Man kocht 4,5 Stunden am Rückfluß, trennt anschließend die Phasen, trocknet die org. Phase und engt sie im Vakuum ein.
Es bleiben 33,9 g der Titelverbindung als rohes Öl zurück.

d)

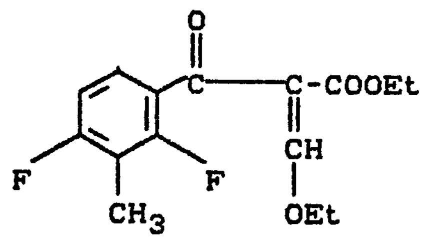

2- (2,4-Difluor-3-methyl-benzoyl)-3-ethoxyacrylsäureethylester

33,9 g roher (2,4-Difluor-3-methyl-benzoyl)-essigsäureethylester, 32 g Orthoameisensäureethylester und 36 g Essigsäureanhydrid werden 2 Stunden auf 150° C erhitzt. Danach wird zunächst im Wasserstrahlvakuum und dann im Hochvakuum bei 130° C Badtemperatur abdestilliert.
Es bleiben 30,7 g der Titelverbindung zurück (Öl).

Beispiel 4

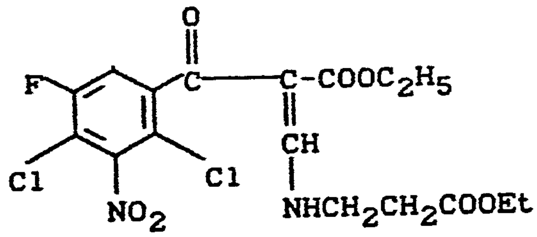

2-(2,4-Dichlor-5-fluor-3-nitro-benzoyl)-3-(2-ethoxycarbonylethylamino)-acrylsäureethylester

38 g Ethoxyacrylester aus Beispiel 1 werden in 150 ml Methylenchlorid vorgelegt. Dazu gibt man eine wäßrige Lösung von 15,4 g 3-Aminopropionsäureethylester-hydrochlorid. Zu dem Zweiphasensystem wird unter gutem Rühren eine Lösung von 8,4 g $NaHCO_3$ in Wasser getropft. Nach Ende des Zutropfens wird noch 2 Stunden gerührt, und anschließend werden die Phasen getrennt.
Die org. Phase wird mit Wasser gewaschen, über $Na_2SO_4$ getrocknet und eingeengt. Der feste Rückstand wird in Diethylether aufgeschlämmt und anschließend isoliert.
Ausbeute: 38 g, Schmelzpunkt 107-8° C.

Beispiel 5

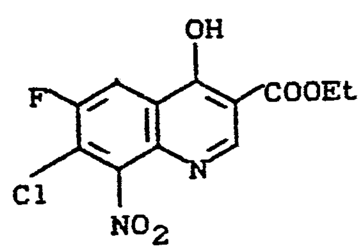

7-Chlor-6-fluor-4-hydroxy-8-nitro-3-chinolincarbonsäure-ethylester

52 g Produkt aus Beispiel 4 und 18,5 g $K_2CO_3$ werden in 160 ml DMF 4 Stunden auf 140°C erhitzt. Anschließend gibt man auf Eis, säuert an und isoliert den ausgefallenen Feststoff. Er wird getrocknet und aus Glykolmethyletheracetat umkristallisiert. Ausbeute: 35,8 g, Schmelzpunkt: 226-8°C(Z).

Beispiel 6

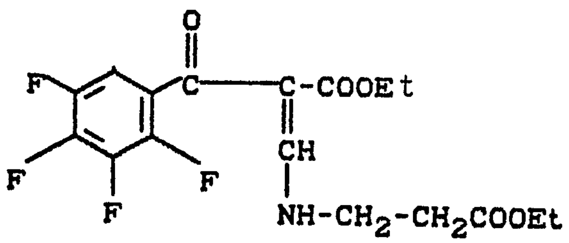

3-(2-Ethoxycarbonyl-ethylamino)-2-(2,3,4,5-tetrafluor-benzoyl)acrylsäureethylester

6,4 g 3-Ethoxy-2-(2,3,4,5-tetrafluor-benzoyl)-acrylsäure-ethylester werden in 30 ml Methylenchlorid vorgelegt. Dazu wird eine Lösung von 3,1 g 3-Amino-propionsäureethylesterhydrochlorid in 12 ml Wasser gegeben. Zu dem Zweiphasengemisch wird unter starkem Rühren eine Lösung von 1,7 g $NaHCO_3$ in 20 ml Wasser getropft. Nach beendetem Zutropfen wird noch 3 Stunden gerührt. Danach trennt man die Phasen, wäscht die org. Phase mit Wasser und trocknet sie über $Na_2SO_4$. Nach Einengen bleiben 7,8 g der Titelverbindung zurück.
Schmelzpunkt: 96-97°C.

Beispiel 7

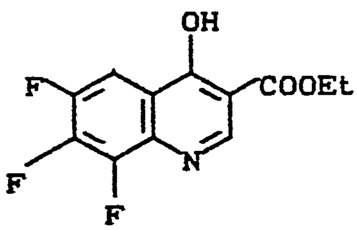

6,7,8-Trifluor-4-hydroxy-3-chinolincarbonsäureethylester

7,7 g Verbindung aus Beispiel 6 und 2,9 g $K_2CO_3$ werden in 36 ml DMF 4 Stunden auf 140-5°C erhitzt. Anschließend gießt man auf Eis, säuert an, trennt den ausgefallenen Feststoff ab und trocknet ihn.
Ausbeute: 4,5 g, Schmelzpunkt: 279-82°C.

Beispiel 8

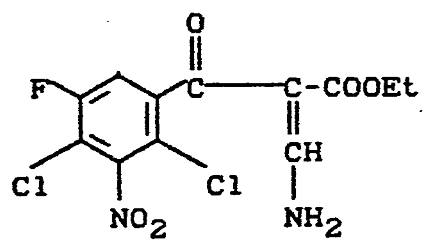

3-Amino-2-(2,4-dichlor-5-fluor-3-nitro-benzoyl)-acrylsäure-ethylester

38 g Produkt aus Beispiel 1 werden in 40 ml Ethanol vorgelegt. Unter Eiskühlung tropft man ein Gemisch aus 8 ml konz. $NH_3$-Lösung in 25 ml Ethanol zu. Man rührt 2 Stunden bei Raumtemperatur und trennt dann die Phasen. Die org.

 

Phase wird mit Wasser gewaschen und getrocknet. Beim Einengen bleiben 38,3 g Titelverbindung zurück. Schmelzpunkt: 113-114° C.

Beispiel 9

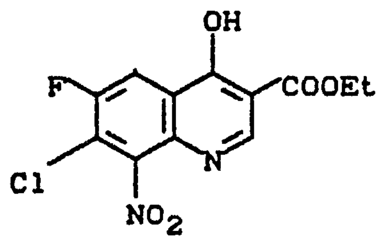

7-Chlor-6-fluor-4-hydroxy-8-nitro-3-chinolincarbonsäure-ethylester

7,0 g Produkt aus Beispiel 8 werden in 100 ml Dioxan vorgelegt. Dazu gibt man portionsweise 4,7 g Kalium-tert.-butylat. Man rührt 24 Stunden bei Raumtemperatur, gießt auf Eis und stellt neutral. Der ausgefallene Feststoff wird isoliert und getrocknet.

Ausbeute: 5,3 g, Schmelzpunkt: 224-7° (Z)

Beispiel 10

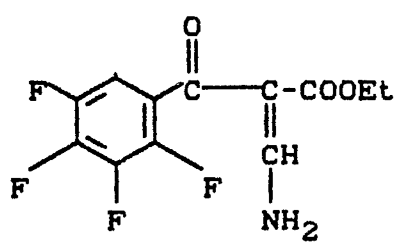

3-Amino-2-(2,3,4,5-tetrafluor-benzoyl)-acrylsäureethylester

12,8 g 3-Ethoxy-2-(2,3,4,5-tetrafluor-benzoyl)-acrylsäure-ethylester werden in 16 ml EtOH vorgelegt. Unter Eiskühlung tropft man ein Gemisch aus 3,3 ml konz. wäßriger $NH_3$-Lösung und 10 ml EtOH zu. Anschließend wird 2 Stunden gerührt. Danach versetzt man mit Eiswasser und saugt den ausgefallenen Feststoff ab.
Ausbeute: 8,7 g, Schmelzpunkt: 118-119° C.

Beispiel 11

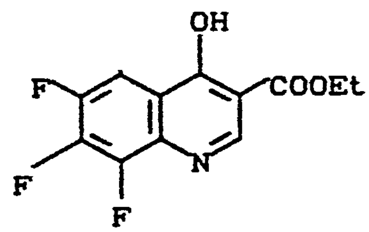

6,7,8-Trifluor-4-hydroxy-3-chinolin-carbonsäureethylester

5,8 g Produkt aus Beispiel 10 werden in 25 ml Dioxan vorgelegt. Bei Raumtemperatur werden 4,8 g Kalium-tert.-butylat portionsweise zudosiert.

Dabei erwärmt sich das Reaktionsgemisch. Man läßt 24 Stunden rühren, gießt dann auf Eis und stellt sauer. Der Feststoff wird isoliert, getrocknet und aus Glykolmonomethylether/Ethanol umkristallisiert.
Ausbeute: 3,1 g, Schmelzpunkt 278° C.

Beispiel 12

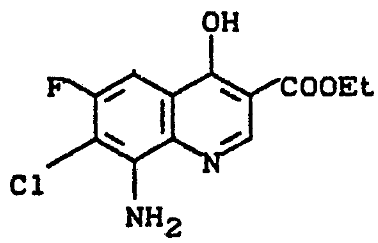

8-Amino-7-chlor-6-fluor-4-hydroxy-3-chinolincarbonsäure-ethylester

9,4 g Produkt aus Beispiel 5 und 19,2 g Eisenspäne werden in 190 ml Essigsäure 1 Stunde auf 80° C erwärmt. Darauf wird kurz auf 110° C erhitzt und heiß filtriert. Danach gibt man zum Filtrat Eiswasser und isoliert den ausgefallenen Feststoff. Nach Trocknen bei 110° C wird aus Glykolmonomethylether umkristallisiert.
Ausbeute: 7,8 g, Schmelzpunkt: 268-70° C.

Patentansprüche

1. Verfahren zur Herstellung von Verbindungen der Formel I

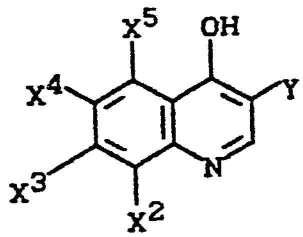

I

in welcher

Y eine Nitrilgruppe, eine Estergruppe $COOR^1$ oder ein Säureamid $CONR^2R^3$ darstellt, wobei $R^1$, $R^2$ und $R^3$ für Wasserstoff oder $C_1$-$C_4$-Alkyl stehen und $R^3$ gegebenenfalls Phenyl sein kann und

$X^2$, $X^3$, $X^4$ und $X^5$ für Wasserstoff, Halogen, Nitro, Cyano, Alkyl mit 1-3 Kohlenstoffatomen, Alkoxy mit 1-3 Kohlenstoffatomen im Alkylrest, Alkylmercapto mit 1-3 Kohlenstoffatomen im Alkylrest, Alkylsulfonyl mit 1-3 Kohlenstoffatomen im Alkylrest, sowie eine gegebenenfalls im Arylrest substituierte Phenylsulfonylgruppe stehen,

dadurch gekennzeichnet, daß man Aminoacrylester der Formel II

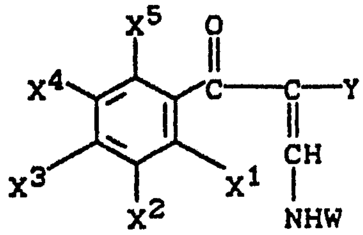

II

in der Y, $X^2$, $X^3$, $X^4$ und $X^5$ die oben angegebene Bedeutung haben, und

$X^1$ Halogen, bevorzugt Fluor und Chlor, eine Nitrogruppe,eine Alkoxy-, Alkylmercapto- oder Alkylsulfonylgruppe mit jeweils 1-3 Kohlenstoffatomen sowie eine Arylsulfonylgruppe bedeutet und

W für Wasserstoff und einen Rest $-CH_2CH_2Z$ steht, wobei

Z für eine Nitrilgruppe, eine Estergruppe $COOR^4$ oder eine Säureamidgruppe $CONR^5R^6$ steht, wobei die Reste $R^4$, $R^5$, $R^6$ Wasserstoff oder $C_1$-$C_4$-Alkyl darstellen und $R^6$ gegebenenfalls Phenyl sein kann,

in einem aprotischen Lösungsmittel in Gegenwart einer Base umsetzt.

2. Verfahren nach Anspruch 1, dadurch gekennzeichnet, daß man die Cyclisierungsreaktion (II) ⟶ (I) in einem Verdünnungsmittel durchführt.

3. Verfahren nach Anspruch 1, dadurch gekennzeichnet, daß man die Cyclisierungsreaktion (II) ⟶ (I) bei Temperaturen von 60 bis 300° C, insbesondere 80 bis 180° C durchgeführt.

4. Verfahren nach Anspruch 1, dadurch gekennzeichnet, daß man als Säurebinder für die Cyclisierungsreaktionen (II) ⟶ (I) Kalium-tert.-butanolat, 1,4-Diaza-bicyclo[2,2,2]-octan (DABCO), 1,8-Diaza-bicyclo[5,4,0]undec-7-en (DBU), Butyl-lithium, Lithiumphenyl, Phenyl-magnesiumbromid, Natriummethylat, Natriumethylat, Natriumhydroxid, Natrium- oder Kaliumcarbonat einsetzt.

5. Verfahren nach Anspruch 4, dadurch gekennzeichnet, daß man für die Cyclisierung (II) ⟶ (I) im Falle W = Wasserstoff zwei Äquivalente Base und im Falle W = $CH_2CH_2Z$ ein Äquivalent einer Base, gegebenenfalls in einem Überschuß von 10 Mol-% an Base, einsetzt.

6. 4-Hydroxy-3-chinolincarbonsäurederivate der Formel I

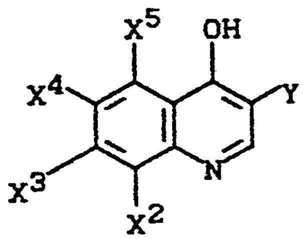

I

in welcher

Y eine Nitrilgruppe, eine Estergruppe $COOR^1$ oder ein Säureamid $CONR^2R^3$ darstellt, wobei $R^1$, $R^2$ und $R^3$ für

Wasserstoff oder $C_1$-$C_4$-Alkyl stehen und $R^3$ gegebenenfalls Phenyl sein kann,

$X^5$ für Wasserstoff, Halogen, Nitro, Cyano, $C_1$-$C_3$-Alkyl, $C_1$-$C_3$-Alkoxy, $C_1$-$C_3$-Alkylmercapto, $C_1$-$C_3$-Alkylsulfonyl, sowie eine gegebenenfalls im Arylrest substituierte Phenylsulfonylgruppe steht,

$X^2$ für Nitro, Cyano, eine Alkyl-, Alkoxy-, Alkylmercapto- oder Alkylsulfonylgruppe mit jeweils 1-3 Kohlenstoffatomen im Alkylteil sowie eine gegebenenfalls im Arylrest substituierte Phenyl sulfonylgruppe steht und

$X^3$ und $X^4$ für Nitro und Halogen, insbesondere Chlor und Fluor stehen.

7. Verbindungen gemäß Formel I nach Anspruch 6, in der Y, $X^2$, $X^3$, $X^4$ und $X^5$ die in Anspruch 6 angegebene Bedeutung haben und $X^2$, $X^3$, $X^4$ und $X^5$ zusätzlich für Amino stehen können.

8. Verbindungen aus der Gruppe bestehend aus

2-(2,4-Difluor-3-methyl-benzoyl)-3-ethoxyacrylsäure-ethylester,
2-(2,4-Dichlor-5-fluor-3-nitro-benzoyl)-3-(2-ethoxycarbonyl-ethylamino)-acrylsäureethylester,
3-(2-Ethoxycarbonyl-ethylamino)-2-(2,3,4,5-tetrafluor-benzoyl)-acrylsäurethylester,
3-Amino-2-(2,4-dichlor-5-fluor-3-nitro-benzoyl)-acrylsäureethylester,
3-Amino-2-(2,3,4,5-tetrafluor-benzoyl)-acrylsäure-ethylester,
8-Amino-7-chlor-6-fluor-4-hydroxy-3-chinolincarbonsäureethylester.

9. Verbindungen aus der Gruppe bestehend aus

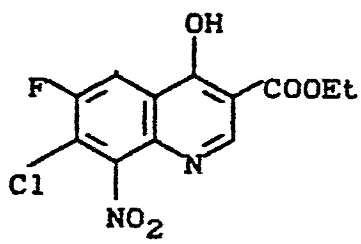

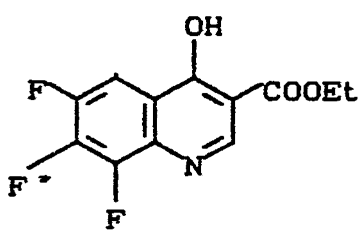

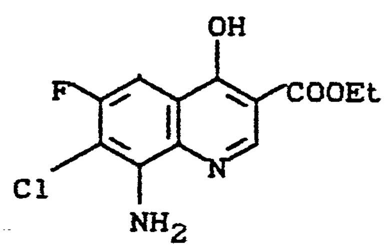

10. Verwendung von Verbindungen der Formel I nach Anspruch 1 zur Herstellung von 1-Alkyl-4-chinolon-3-carbonsäuren.

0245690

Europäisches Patentamt

# EUROPÄISCHER RECHERCHENBERICHT

Nummer der Anmeldung

EP 87106123.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 168 733 (BAYER)<br>* Ansprüche 1-3,5,6 *<br>-- | 1-4 | C 07 D 215/56<br>C 07 C 101/34<br>C 07 C 69/738 |
| A | EP - A2 - 0 168 737 (BAYER)<br>* Ansprüche 1-3,5,6 *<br>-- | 1-4 | |
| A | EP - A2 - 0 176 846 (BAYER)<br>* Ansprüche 1,11 *<br>-- | 1,8,<br>10 | |
| A | DE - A1 - 2 854 558 (VEB JENA PHARM)<br>* Ansprüche 1,3,12 *<br>---- | 1,6 | |
| | | | RECHERCHIERTE SACHGEBIETE (Int. Cl.4) |
| | | | C 07 D 215/00<br>C 07 C 101/00<br>C 07 C 69/00 |

Der vorliegende Recherchenbericht wurde für alle Patentansprüche erstellt.

| Recherchenort | Abschlußdatum der Recherche | Prüfer |
|---|---|---|
| WIEN | 11-08-1987 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82